# EUROPEAN PATENT SPECIFICATION

(11) **EP 1 787 596 B1**
(45) Date of publication and mention of the grant of the patent: **08.08.2012**
(21) Application number: 06024122.1
(22) Date of filing: 21.11.2006
(51) Int. Cl.: A61B 18/14

(54) **A bipolar electrosurgical sealing instrument having an improved tissue gripping device**
Ein bipolares elektrochirurgisches Instrument zum Verschließen von Gewebe mit einem verbesserten Gewebehalter
Un instrument électrochirurgical bipolaire pour souder du tissu avec un dispositif à pincer le tissu amélioré

(30) Priority: 22.11.2005 US 285425
(43) Date of publication of application: 23.05.2007
(73) Proprietor: Covidien AG, 8212 Neuhausen am Rheinfall (CH)
(72) Inventor: Tetzlaff, Philip M., Lafayette Colorado 80026 (US); Artale, Ryan, Boulder, CO 80305 (US)
(74) Representative: HOFFMANN EITLE

(56) References cited:
- EP-A- 0 589 453
- US-A1- 4 671 274
- US-A1- 5 217 460
- US-A1- 5 797 927
- US-A1- 5 902 301
- US-A1- 2003 181 910

## Description

### BACKGROUND

### 1. Technical Field

The present disclosure relates to an electrosurgical instrument for permanently closing epidermis and/or body tissue sections in a human or animal, and more particularly to a bipolar electrosurgical instrument having an improved gripping device. The gripping device grips, pulls together and.seals epidermis and dermis tissue sections by applying a combination of pressure and electrosurgical current, while not disturbing any subcutaneous adipose tissue sections. The gripping device could also be used to oppose and fuse other types of tissue.

### 2. Background of the Related Art

Coagulation instruments are known in the art. Coagulation is defined as a process of desiccating tissue wherein the tissue cells are ruptured and dried. Vessel sealing is also known in the art. Vessel sealing is the process of liquefying the collagen in the tissue so that it crosslinks and reforms into a fused mass. Coagulation of vessels is thus sufficient to permanently close them. Small and large vessels may be sealed to assure a permanent closure.

Typically, to seal vessels and/or tissue the selected tissue or vessel is placed between a first seal surface and a second seal surface of an instrument. A handle with a first looped member and a second looped member is gripped. The handle controls the seal surfaces to grip and hold the selected tissue or vessel between the first seal surface and the second seal surface. Thereafter, an electrical current is introduced through the instrument. The current is introduced by actuating an actuator to direct the current through the first seal surface through the tissue or vessel and through the second seal surface. This electrical current and the pressure imparted to the selected vessel (or tissue) causes coagulation or a liquefying of the collagen in the tissue so that the liquefied tissue is cross-linked and the tissue reforms into a fused mass. Thereafter, the seal is completed and the instrument is withdrawn by the surgeon.

Full depth skin incisions usually involve a laceration or injury where a tissue section having an epidermis section, a dermis section and a subcutaneous adipose section are all cut. Some so called "full depth" cuts or so called "full depth incisions" extend into the skin a predetermined depth. The depth is through the top epidermis section through the intermediate dermis section and also sometimes through the bottom subcutaneous adipose section.

The outermost epidermis is made from a stratified squamous epithelium. These stratified squamous epithelium cells typically can withstand an amount of stress and are easier to manipulate relative to other layers. The subcutaneous adipose section, however, is made from a loose connective tissue. This loose connective tissue is more difficult to manipulate relative to the previously described stratified squamous epithelium. The cells of this type of loose connective tissue are generally separated by quite some distance relative to the previously described stratified squamous epithelium and are separated by a gel-like or gelatinous substance. The gel-like or gelatinous substance is primarily made up of collagenous and elastic fibers.

This gelatinous substance is difficult to manipulate and will move and slip around if placed between, for example, the jaws of a bipolar instrument. This does not contribute to treatment of the epidermis of the full depth skin incisions using electrical current and pressure to cause coagulation or a liquefying of the epidermis so that that the liquefied tissue is cross-linked and the tissue reforms into a fused mass. A known problem is that in these applications (if it is even attempted) the subcutaneous adipose section will slide from the jaws and thus drag other tissue sections with it. This gelatinous substance of the subcutaneous adipose section impedes alignment of any lacerated edges of the epidermis and skin sections that are desired to be sealed. Thus, a known problem in the art is that the surgeon has difficultly applying the electrical current successfully to the epidermis section of such a full depth skin incision, and the lacerated edges are difficult to align and maintain in alignment once aligned between the jaws for treatment.

Accordingly, there is a need for an improved gripping device for a bipolar sealing device for aligning a first lacerated edge of a first tissue component with a second lacerated edge of another tissue component. There is also a need for such a gripping device that holds and prevents the lacerated tissue component from traversing out from any alignment between the first and second tissue components. There is also a further need for such a gripping device for a bipolar sealing device that only grips a preselected optimal amount of the tissue and does not disturb other tissue in order to align the first and the second lacerated edges for coagulation.

EP 0 589 453 discloses a bipolar surgical forceps, wherein jaws of the forceps have serrated edges for securing tissue therebetween. The pre-amble of claim 1 is based on this document. US 2003/0181910 disclose bipolar electrosurgical foreigns with non-conductive stop markers that are generally L-shaped.

### SUMMARY

According to a first aspect of the present disclosure, and as recited in claim 1, there is provided a bipolar electrosurgical instrument. The bipolar electrosurgical instrument has inner and outer members each having a respective jaw member disposed at a distal end thereof. Each jaw member has right and left side surfaces and seal surface. The seal surfaces are adapted to connect to an electrical energy source such that the seal surfaces are capable of conducting bipolar energy therebetween. The inner and outer members are movable from a first position where the jaw members are disposed in spaced relation-relative to one another to a second position where the jaw members are closer to one another for grasping tissue. The instrument further has a first gripping device disposed on at least one of the right and left side surfaces of one of the jaw members. The first gripping device includes tines extending therefrom dimensioned to engage and hold tissue when the jaw members are moved from the first position to the second position.

According to a preferred aspect of the present disclosure, the instrument also has each of the tines with a free gripping end. The free gripping end is suitable to manipulate an epidermis section of tissue but not disturb a subcutaneous adipose tissue layer when the outer and inner members move from the first position to the second position.

According to a further preferred aspect of the present disclosure, the instrument also has a plurality of tines disposed on at least one of the right and left side surfaces of one of the jaw members. The tines are dimensioned to engage and hold tissue when the jaw members are moved from the first position to the second position. The tines form an epidermis gripping zone. The epidermis gripping zone is suitable to grasp and retain an epidermis section of tissue and a dermis section of tissue between the seal surfaces but not contact any subcutaneous adipose tissue layer.

According to another aspect of the present disclosure, not forming part of the claimed invention , there is provided a method of sealing tissue. The method has the steps of providing a bipolar instrument including inner and outer members each having a respective jaw member disposed at a distal end thereof. Each jaw members include right and left side surfaces and a seal surface. The seal surfaces are adapted to connect to an electrical energy source such that the seal surfaces are capable of conducting bipolar energy therebetween. The instrument has a first gripping device disposed on at least one of the right and left side surfaces of one of the jaw members. The first gripping device includes a plurality of tines extending therefrom dimensioned to engage and hold tissue when the jaw members are moved from the first position to the second position. The method has the steps of moving the inner and outer members from the first to second positions and engaging tissue with the gripping device. The method further has the steps of closing the first and second jaw members about tissue and activating an electrical energy source to seal tissue between the seal surfaces.

### BRIEF DESCRIPTION OF THE DRAWINGS

FIG. 1 is a view of a first tissue component with a first epidermis section, a first dermis, and a first subcutaneous adipose section adjacent to a second tissue component with a second epidermis section, a second dermis, and a second subcutaneous adipose section;
FIG. 1A is a view of a bipolar sealing instrument for tissue fusion of the present disclosure grasping and sealing the first epidermis section with the second epidermis section and not grasping any first and second subcutaneous adipose sections that disturb alignment;
FIG. 2 is a close up view of the jaws of the bipolar instrument for tissue fusion having a number of tines;
FIG. 3 is a perspective view of a bipolar instrument for tissue fusion;
FIG. 3A is a close up view of a first tine;
FIG. 3B is a close up of an alternative embodiment of the first tine of Fig. 3A;
FIG. 3C is a close up of an alternative embodiment of the first tine of Fig. 3A;
FIG. 3D is a close up of an alternative embodiment of the first tine of Fig. 3A;
FIG. 3E is a close up of an alternative embodiment of the first tine of Fig. 3A;
FIG. 3F is a close up of an alternative embodiment of the first tine of Fig. 3A;
FIG. 4 is a perspective view of the bipolar sealing instrument shown partially exploded;

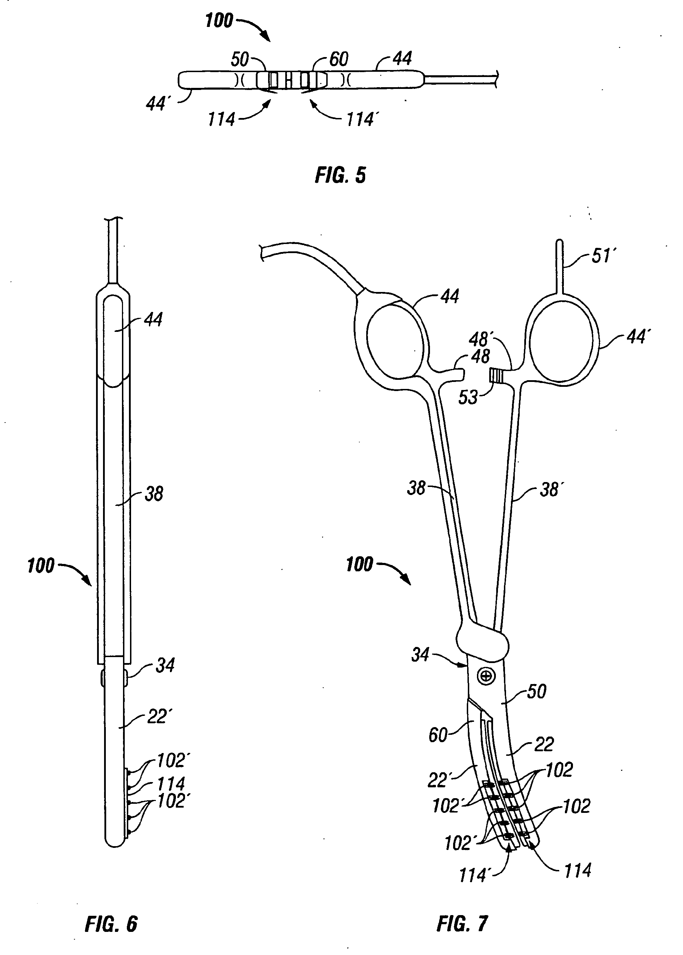

FIG. 5 is a front view of the bipolar sealing instrument of Fig. 3;
FIG. 6 is a first lateral side view of the bipolar sealing instrument of Fig. 6.

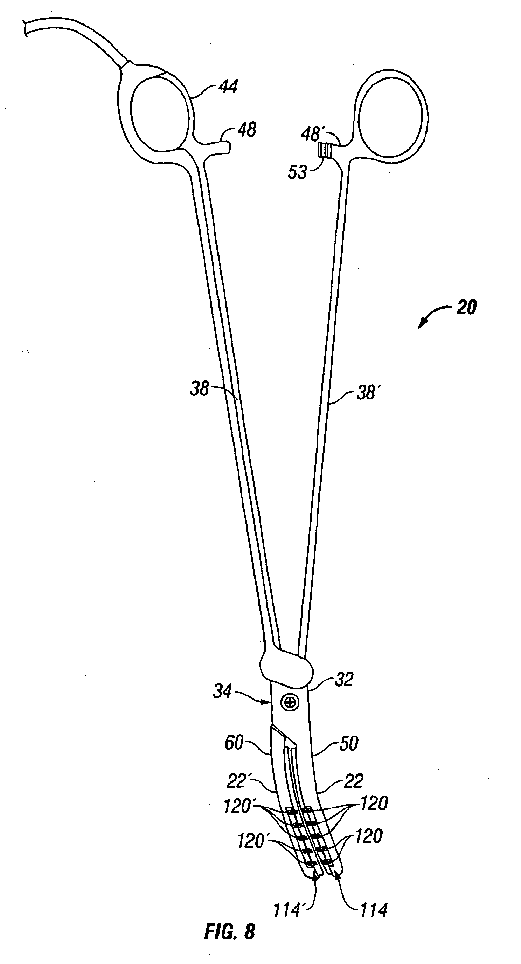

FIGS. 7 and 8 are top views of the bipolar sealing instrument of Fig. 6; and
FIG. 9 shows an alternative embodiment of the bipolar sealing instrument of Fig. 6 having gripping tines extending from a distal most location of the outer and the inner jaws.

### DETAILED DESCRIPTION

In the drawings and in the description which follows, the term "proximal", as is traditional, will refer to the end of the apparatus which is closest to the operator, while the term "distal" will refer to the end of the apparatus which is furthest from the operator. It should be appreciated that these designation form no limitations on the present disclosure whatsoever.

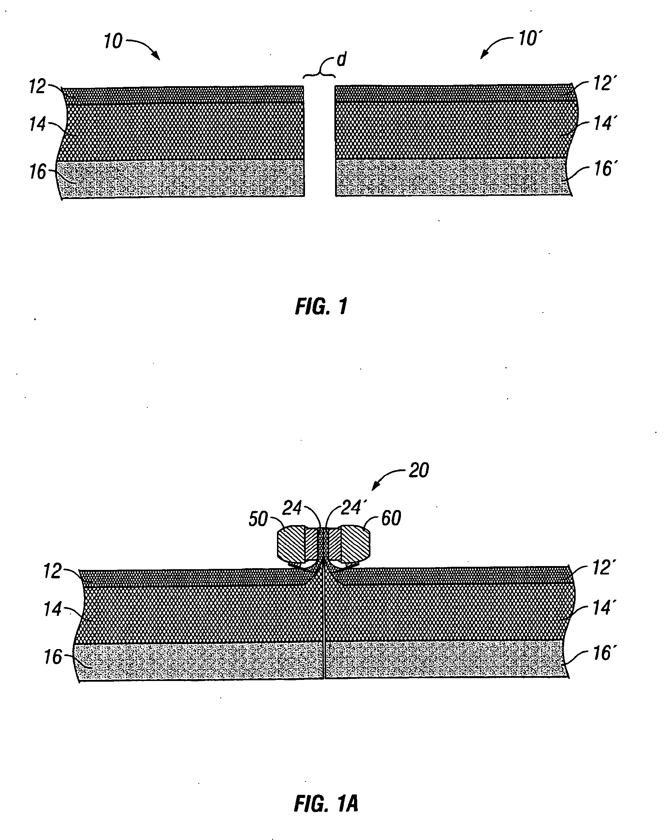

Fig. 1 shows a cross sectional view of a full depth incision with a first tissue component 10 and a second tissue component 10'. The first tissue component 10 has a first epidermis section 12, a first dermis section 14, and a first subcutaneous adipose section 16. Such a full depth incision is defined as a separation of the skin, into two portions, by a sharp object through the application of an acutely directed force that extends at least though the epidermis section 12 and the dermis section 14 of the first tissue component 10.

The apparatus of the present disclosure has an improved skin grasping feature. The feature is advantageous over known surgical instruments because the apparatus and method of the present disclosure can grasp and seal or fuse the first tissue component 10 and the second tissue component 10' in an improved manner over the art because the apparatus only grasps at a preselected portion of the skin.

The second tissue component 10' also shown in cross section in Fig. 1 has a second epidermis section 12', a second dermis section 14', and a second subcutaneous adipose section 16'. As can be understood from the figures, the tissue components 10 and 10' are shown as separated from one another by a distance marked by reference letter "d" for illustration purposes. However, in actuality some tissue components may be separated by a slight difference or gap and other tissue sections may be separated by another or different distance. Further, in another embodiment some of the tissue components (such as the first and second subcutaneous adipose sections 16 and 16') may be still connected to one another while some other tissue components may be fully separated from one another by the distance "d". Various configurations are possible and are all within the scope of the present disclosure.

Fig. 1A shows a cross sectional view of a first tissue component 10 and a second tissue component 10' and bipolar sealing instrument of the present disclosure generally represented by reference number 20. The bipolar sealing instrument 20 has a jaw member 50 with a first seal surface 24 and a jaw member 60 with a second seal surface 24'. The jaw members 50, 60 move from an opened position where the jaw members 50, 60 are separated relative to one another to a closed position wherein the first seal surface 24 is closer to the second seal surface 24'.

The first and the second jaw members 50, 60 are selectively positionable to grasp the tissue (i.e., the first tissue component 10 and the second tissue component 10') along the lateral edges between the jaw members 50, 60. One significant advantage of the present disclosure over other known devices is that bipolar sealing instrument 20 aligns only the desired tissue components between the seal surfaces 24, 24' as shown in Fig. 1A.

Once aligned, the user selectively activates the bipolar sealing instrument 20 to energize the seal surfaces 24, 24' to apply bipolar energy across the seal surfaces 24 and 24'. One example of a sealing instrument is disclosed in commonly-owned United States Patent Nos. 6,187,003 , 6,726,686 B2, and 6,352,536 to Buysse, et al. Other instruments are disclosed in commonly owned PCT Patent Publication No. WO 2002/080797, U.S. Patent No. 6,511,480 and U.S. Patent No. 6,277,117.

It has been observed with a skin sealing application that it is desirable to only grip the first epidermis section 12 with the first dermis section 14 and the second epidermis section 12' with the second dermis section 14'. As mentioned above, it has also been observed that it is not desirable to grip any portions of the first subcutaneous adipose section 16 or second subcutaneous adipose section 16' for coagulating since this may affect the success of the tissue seal. More particularly, it has been observed that the first and second subcutaneous adipose sections 16, 16' have cells which are typically not disposed close to one another relative to the other dermis sections 12, 12', 14, 14'. Often these cells are separated by a loose gel-like connective tissue which makes the first and/or second subcutaneous adipose sections 16, 16' difficult to handle and/or manipulate between the jaw members 50, 60.

Further, grasping the first and/or second subcutaneous adipose sections 16, 16' with the first and/or second epidermis sections 12, 12' often disturbs or misaligns the instrument's orientation which in turn may cause misalignment of the tissue structures.

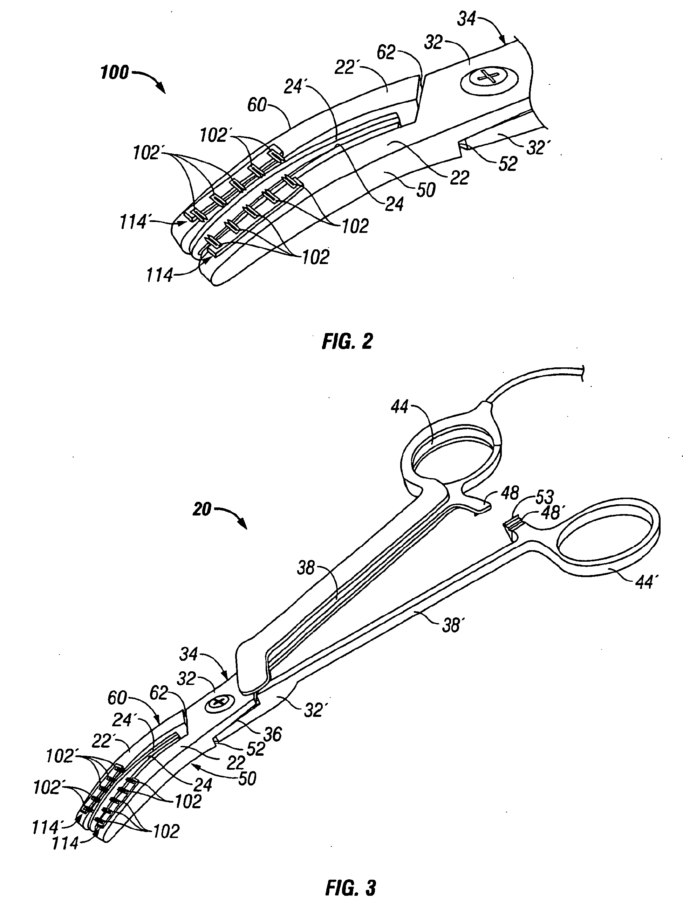

Figs. 1A and 2 show the bipolar sealing instrument 20 of the present disclosure with a gripping device 100. The gripping device 100 assists gripping the first epidermis section 12 and the first dermis section 14 with the second epidermis section 12' and the second dermis section 14'. The gripping device 100 specifically does not grip or otherwise engage the first and second subcutaneous adipose sections 16 and 16'.

Fig. 2 shows the bipolar sealing instrument 20 with the gripping device 100. The gripping device 100 includes an array of tines 102 or prongs disposed on at least one side 22 of the bipolar sealing instrument 20. The tines 102 are typically thin, pointed or needle-like projections which extend inwardly (i.e., toward tissue engaging surfaces 24, and 24') from the bipolar sealing instrument 20 and are configured to grasp epidermis section 12, 12' of a deep skin incision. Alternatively, the gripping device 100 may have a series of protrusions, knobs, teeth, branches, connected branches, or members each collectively forming an aligned pattern on one or both sides of the bipolar sealing instrument 20 for gripping.

The gripping device 100 includes a pair of opposing comb-like gripping members 114 and 114' disposed on jaw members 50 and 60, respectively. More particularly, each gripping member 114 and 114' is disposed on the respective outer surface 22 and 22' of each jaw member 50 and 60 such that a series of comb-like or tine elements 102 and 102' common to each of the gripping members 114 and 114', respectively, oppose one another for gripping and holding tissue. As best shown in Fig. 2, the plurality of tines 102 disposed on surface 22 is generally offset relative to the corresponding plurality of tines 102' disposed on surface 22' along the length of the jaw members 50 and 60. It is envisioned that this promotes better grasping of tissue structures. Alternatively, the tines 102 and 102' may register with one another along the length of the jaw members 50 and 60 depending upon a particular purpose or to achieve a particular surgical result.

The gripping members 114, and 114' may be dimensioned to extend partially along the entire length of each jaw member, e.g. 50, or may be dimensioned to extend along the entire length of the same. Alternatively, a series of gripping elements 114 may be staggered across the length of the jaw member 50 with an opposing series of gripping elements 114' staggered along the opposite jaw member 60.

The gripping device 100 will contact the relevant epidermis 12, 12' and/or dermis 14, 14' sections and firmly grasp only the relevant epidermis/dermis 12, 12', 14, and 14' sections while specifically not contacting other tissue sections or components that may disturb the orientation of the relevant epidermis and dermis tissue sections 12, 12', 14, and 14'. This is particularly advantageous to assist with the tissue sealing using RF electrosurgical energy. The gripping device 100 thus allows for a more precise operation of the bipolar sealing instrument 20.

In one embodiment, each of the tines 102, 102' may be substantially "U" shaped and arranged in rows of five; however any number of tines 102 may be used for the bipolar sealing instrument 20 of the present disclosure.

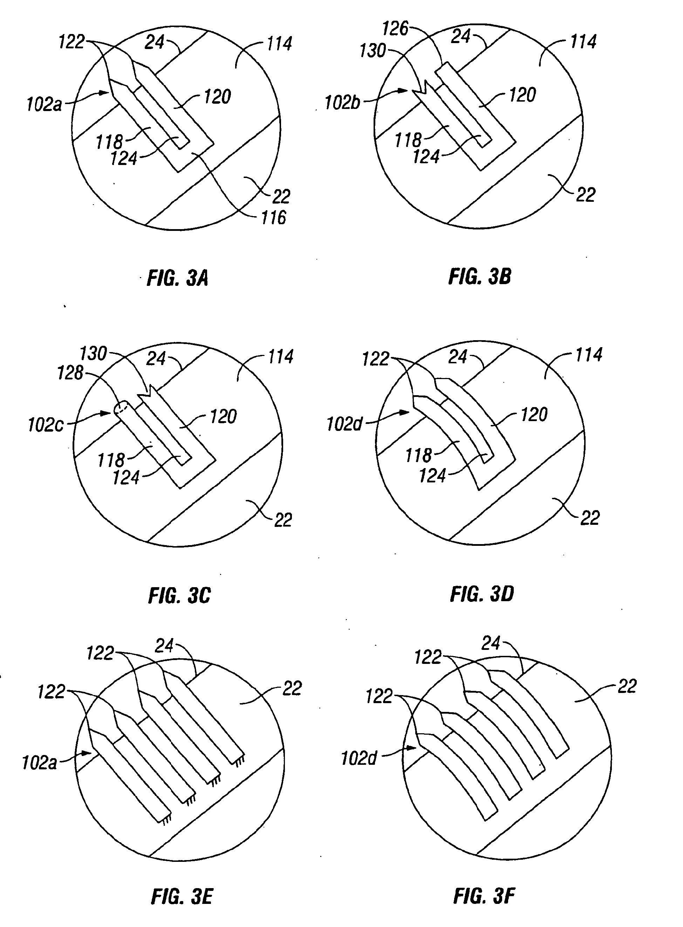

Fig. 3A shows an enlarged view of an alternative configuration for the tine 102 (and/or 102'). The tine 102 of this particular embodiment is configured in a "U" shape with a base portion 116. The base portion 116 connects to gripping member 114 and includes two arms 118, 120 which extend from the base 116. Each arm 118 and 120 is spaced relative to one another and includes a point 122 at a distal end thereof for engaging the tissue. However, the end 122 is not limited to this configuration. In other embodiments, the ends 122 may be rectangular (end 126), cylindrical (end 128) or include a plurality of points (end 130) as seen in Figs. 3B and 3C. As shown in Figs. 3B and 3C, each end 122 is disposed on a single base may have differently-shaped ends 126, 128 and 130 respectively. Various configurations of the tines 102 are possible and within the scope of the present disclosure and have been shown in the various figures 3A through 3F as 102a through 102f.

Referring to Fig. 3A, in one embodiment, the arms 118 and 120 are straight planar members. Alternatively, the arms 118 and 120 may not be straight and instead have a curvature as shown in Fig. 3D.

Referring to Fig. 3E, in one embodiment, the tines 102e may extend simply from the side 22 without any gripping member 114. Moreover, the tines 102a may be configured in a longitudinal or "I" shaped configuration. In this embodiment, each of the tines 102 may simply be spaced from one another by a predetermined distance along the side 22 as shown in Fig. 3E. Again, alternatively as shown the tines 102 in Fig. 3F, may have a curvature to assist with grasping the relevant tissue sections and to facilitate treatment.

Turning back to Fig. 3 which shows a perspective view of one particularly simple bipolar sealing instrument 20 in an assembled state, the bipolar sealing instrument 20 has an inner member 32' and an outer member 32. The members 32 and 32' are connected through an open lockbox 34 which has a gap 36 between flanges. The terms "inner" and "outer" are used to distinguish the members 32 and 32', and their component parts, according to the members' respective positions with respect to the open lockbox 34, however the bipolar sealing instrument 20 is not limited to any such orientation and the inner member 32' and the outer member 32 may have other configurations. The inner member 32' is fitted generally within the inner surfaces of the open lockbox 34 and is captured between the flanges. The outer member 32 generally forms the outside surfaces of the open lockbox 34. Details relating to one envisioned lockbox 34 are disclosed in commonly owned United States Patent No.: 6,187,003.

The inner member 32' has an inner shank 38' which is operatively associated with the inner jaw 60 at a distal end thereof and has an inner ring handle 44' at a proximal end thereof. Similarly, the outer member 32 has an outer shank 38 at a distal end thereof which operatively connects to the jaw member 50, and an outer ring handle 44 at a proximal end thereof. The inner and the outer jaw members 60, and 50 are designed to grasp tissue between the opposing seal surfaces 24, 24'.

Each shank, 38 and 38', has a respective ratchet stub or interlocking member 48 and 48'. Ratchet teeth 53 are designed to interlock in a manner that holds the inner and outer shank, 38 and 38', in position. The shanks 38 and 38' are deflected in the manner of a cantilever spring when the jaw members 50, 60 are forced together by the surgeon. The deflection of the shanks 38 and 38' may produce a spring restoring force that can be opposed by interlocking the ratchet stubs or interlocking members 48, 48'.

The proximal end of the jaw member 50 also includes a rectangular step 52 disposed on an inner facing surface of jaw member 50 proximal sealing surface 24. The rectangular step 52 may be made from a thermally non-conductive material, or alternatively has a thermally non-conductive material connected thereto. The rectangular step 52 with the thermally non-conductive material does not conduct or interfere with the application of any RF electrosurgical energy to the first seal surface 24.

The proximal end of the jaw member 50 also includes rectangular step 62 disposed on an inwardly facing surface of jaw 60 proximal sealing surface 24' and complementary to step 52. The rectangular step 62 also is likewise made from a thermally non-conductive material, or alternatively has a thermally non-conductive material connected thereto. Rectangular step 62 does not conduct or interfere with the application of any RF electrosurgical energy to the seal surface 24.

In operation, the gripping members 114, 114' contact the first and the second epidermis section 12, 12' and the first and the second dermis sections 14, 14' before the sections are grasped by the inner and the outer jaw members 50, 60. The gripping members 114, 114' will then contact and move the desired tissue into an alignment so the first epidermis section 12 and the second epidermis section 12' align. Thereafter, the first dermis portion 14 and the second dermis portion 14' align. The first dermis portion 14 and the second dermis portion 14' are forced in a complementary direction between the jaw members 50, 60. The desired tissue sections are introduced between the first sealing surface 24 and the second sealing surface 24' for sealing by the RF electrosurgical energy.

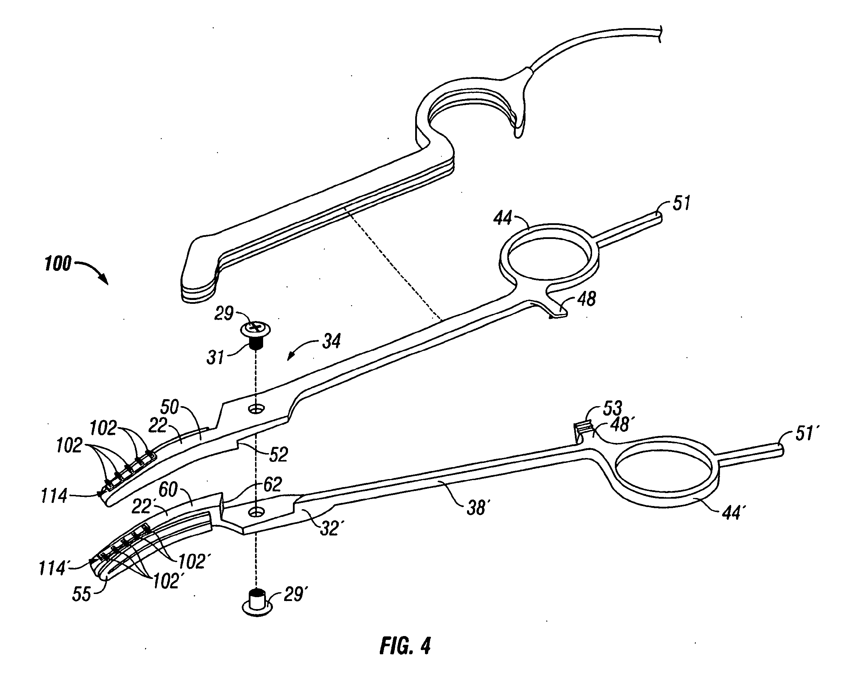

Fig. 4 shows the inner and outer member 32', and 32. Each is connected to a pole of a bipolar electrosurgical generator (not shown). Electrical connectors 51, 51' and are located on the ring handles 44 and 44' to provide a convenient point of connection with a suitable coupling. The inner and the outer members 32' and 32 are formed of an electrically conductive material, such as a stainless steel. The exposed surfaces of the members 32', 32, except for the connectors 51, and 51' and the seal surfaces 24 and 24', are spray coated with an insulating material. The characteristics of the bipolar electrosurgical current are determined by the design of the electrosurgical generator. Moreover, it is envisioned that other open forceps designs are also contemplated wherein the electrical connections are disposed through a single shank member and the different bipolar portions are electrically communicated through the pivot assembly. For example, commonly owned United States Patent No. 6,277,117 and United States Patent Publication Nos. 20050137592 and 20030199869 disclose different configurations or forceps which may be designed for use with the present disclosure.

The generator (not shown) has an output wherein the peak-to-peak voltage will not exceed 130 Volts. This is because higher voltages can cause sparking. This sparking results in localized burning of tissue which may result in a failure of the tissue weld. The generator capable of producing high frequency output current of at least two amps (RMS). High electrical current is one aspect of the present disclosure because it heats the tissue sufficiently to melt the collagen and thus fuse the first and second epidermis sections 12, 12'. Notably, lower electrical currents will often produce weak tissue welds with low bursting strength. During operation, the bipolar sealing instrument 20 is used to grasp tissue between the seal surfaces 24 and 24' using the array of tines 102, 102'. The surgeon squeezes the ring handles 44 and 44' together, causing pressure to be applied to the tissue. The shanks 38 and 38' are designed to maintain closure force of about 3 kg/cm² to about 16 kg/cm² between jaw members 50 and 60.

The magnitude of pressure exerted on the tissue by the seal surfaces 24, 24' is one aspect in assuring a proper surgical outcome. Tissue pressures within a working range of about of 3 kg/cm² to about of 16 kg/cm² and, within a working range of 7 to 13 kg/cm² have been shown to be effective for sealing the desired first epidermis section 12 and the second epidermis section 12'. Tissue pressures within the range of about 4 kg/cm² to about 6.5 kg/cm² have proven to be particularly effective in sealing tissue bundles.

The ratchet teeth 53 are interlocked at the appropriate ratchet setting, depending on the skin type and skin thickness. Bipolar electrosurgical current is applied through the instrument and the first and the second epidermis sections 12, 12' are sealed. Preferably, the jaw members 50, 60 include one or more stop members (not shown) which provide a tissue gap of about 0.001 inches (0.03mm) to about 0.006 inches (0.2mm) between the vessel sealing surfaces 24, 24' when the jaw members 50, 60 are closed about tissue. This is an important mechanical factor to promote tissue sealing.

The jaw members 50, 60 have a structure and cross-section that resist bending under load. Thus the inner and the outer shanks 38', 38 act as a cantilever supported beam once the seal surfaces 24, 24' are closed. The length of this idealized cantilever beam extends from a lockbox screw 31 to the location of the respective interlocking members 48, 48'. It is possible to model each shank 38', 38 as a cantilever spring having a spring constant such that each ratchet position on the teeth 53 is designed to transmit a particular closure force to the jaw members 50 and 60 against the action of the restoring force of the cantilever spring. Preferably, the closure force is produced and maintained within the above working range of 3 kg/cm² to 16 kg/cm².

The open lockbox 34 has the function of providing a pivoting joint for the inner and the outer shanks 38' and 38. In addition, the flanges provide lateral support to help maintain alignment of the jaw members 50 and 60. Closed lockbox designs are typically used and the outer member 32 is completely captured through a slot in the inner member 32'.

The electrically insulated pivot in the present disclosure has a shoulder washer 29 supporting the lockbox screw 31. The shoulder washer 29 is composed of an electrically insulating material that prevents a short circuit between the members 32 and 32'.

Referring now to a distal portion of the bipolar sealing instrument 20, each sealing surface 24, 24' has a radiused edge. In addition, a taper on the seal surface 24, 24' permits a relatively constant pressure on the tissue along the length of the seal surfaces 24, 24'. The width of the seal surfaces 24 and 24' is adjusted, in certain embodiments, wherein the closure force divided by the width is approximately constant along the length.

In one embodiment, the seal surfaces may have a stop member 55, made from insulating material with the stop maintaining a minimum separation of at least about 0.001 inches (0.03mm) between the seal surfaces 24 and 24', as shown in FIG. 4. The stop member 55 reduces the possibility of short circuits between the seal surfaces 24 and 24' as well as promotes tissue sealing.

In use the surgeon positions the forceps to engage the first epidermis section 12 with the first dermis section 14 and the second epidermis section 12' with the second dermis section 14' and they are compressed between the opposable seal surfaces 24 and 24'. The opposable seal surfaces 24, 24' come together in aligned opposition due to the alignment action of the open lockbox 34. The surgeon further deflects the shanks 38, 38' of the members 32, 32' to engage the ratchet stubs 48 and 48'. The engagement of the ratchet stubs 48, 48' hold the shanks 38, 38' in their deflected positions to provide a constant spring force that is transmitted as a closure force to the jaw members 50 and 60.

An electrosurgical generator (not shown) is connected to the bipolar sealing instrument 20 through connectors 51, 51' (Fig. 4) on the ring handles 44, 44'. An electrical switch is used to close a circuit between the generator and the bipolar sealing instrument 20. Alternatively, both connectors 51, 51' may be operatively connected to extend through ring handle 44 as shown.

The switch may be a footswitch such as Valleylab's catalog number E6009, available from Valleylab Inc., Boulder Colo. The electrosurgical current flows through an electrically conductive path on each of the inner and outer members 32, 32' between its respective electrical connector, 51' or 51, and the respective seal surfaces, 24, 24'. An electrically insulating coating substantially covers each member 32, 32' to protect the surgeon against electrical arcs. An insulating sheath 58 may also be used to cover the members or the component parts thereof, i.e., the handles, the shanks and the outer surfaces (non-opposing surfaces) of the jaw members 50, 60.

It is envisioned that the outer surface of the jaw members 50, 60 may include a nickel-based material, a coating, a stamping, or a metal injection molding which is designed to reduce adhesion between the jaw members 50, 60 (or components thereof) with the surrounding tissue during activation and sealing. Other components such as the shanks 38, 38' and the ring handles 44, 44' are coated with the same or a different "non-stick" material. The materials are of a class of materials that provide a smooth surface to prevent mechanical tooth adhesions.

It is also contemplated that the sealing surfaces 24, 24' of the jaw members 50, 60, respectively, may be manufactured from such "non-stick" materials as a nickel-chrome, a chromium nitride, a Med Coat 2000 manufactured by Electrolizing Corporation of Ohio, Inconel 600, tin-nickel, or any alloys thereof. High nickel chrome alloys and Ni200, Ni201 (about 100% Ni) may be made into electrodes or sealing surfaces by metal injection molding, stamping, machining or any like process.

These materials have an optimal surface energy for eliminating sticking due in part to surface texture and susceptibility to surface breakdown due electrical effects and corrosion in the presence of biologic tissues. These materials exhibit superior non-stick qualities over stainless steel and should be used on the sealing instrument 20 in areas where the exposure to pressure and RF energy can create localized "hot spots" more susceptible to epidermis adhesion. Reducing the amount of skin that "sticks" during sealing improves the overall efficacy of the instrument. The sealing surfaces 24, 24' may also be "coated" with one or more of the above materials to achieve the same result, i.e., a "non-stick surface". For example, Nitride coatings (or one or more of the other above-identified materials) may be deposited as a coating on another base material (metal or nonmetal) using a vapor deposition manufacturing technique.

One particular class of materials disclosed herein has demonstrated superior non-stick properties and, in some instances, superior seal quality. For example, nitride coatings which include, but not are not limited to: TiN, ZrN, TiAIN, and CrN are some materials used for non-stick purposes. One is disclosed in commonly owned United States Patent Publication No 2003 0199869.

CrN has been found to be useful for non-stick purposes due to its overall surface properties and performance. Other classes of materials have also been found to reduce overall sticking. High nickel/chrome alloys with a Ni/Cr ratio of approximately 5:1 significantly reduce sticking in bipolar instrumentation. A non-stick material in this class is Inconel 600. Bipolar instrumentation having electrodes made from or coated with Ni200, Ni201 (about. 100% Ni) also showed improved non-stick performance over other bipolar stainless steel electrodes.

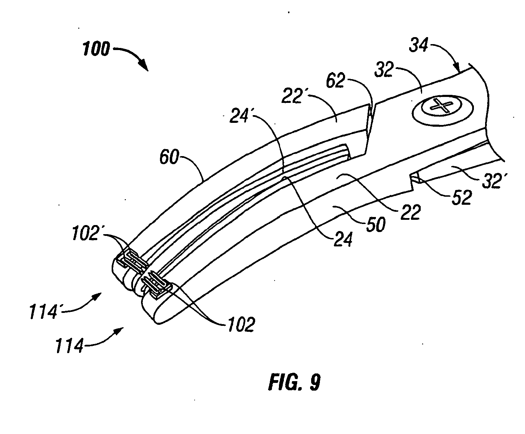

Referring now to Fig. 9, there is shown an alternative embodiment of the bipolar sealing instrument 20. In this embodiment, the bipolar sealing instrument 20 has the gripping tines 102, 102' extending longitudinally from the distal most portion of the inner and the outer jaws 50, 60. The gripping tines 102, 102' extend from this distal most location of the inner and the outer jaws 50, 60 to assist with dissection of tissue. Alternatively, the gripping tines 102, 102' may also assist with the manipulation and gripping of thin tissue for sealing by the bipolar sealing instrument 20. The gripping tines 102, 102' at this longitudinal distal most location of the bipolar sealing instrument 20 may have any length conducive to gripping, manipulating and/or dissecting tissue.

It is to be understood that the above described embodiments are only illustrative of the application of the principles of the present disclosure. Numerous modifications and alternative arrangements may be devised by those skilled in the art.

## Claims

1. A bipolar electrosurgical instrument (20) comprising:
inner and outer members (32', 32) each having a respective jaw member (50, 60) disposed at a distal end thereof, each of said jaw members including outer right and left side surfaces (22, 22') and a seal surface, said seal surfaces (24, 24') adapted to connect to an electrical energy source such that said seal surfaces are capable of conducting bipolar energy therebetween;
said inner and outer members movable from a first position wherein said jaw members are disposed in spaced relation relative to one another to a second position wherein the jaw members are closer to one another for grasping tissue; and
a first gripping device (100) including a plurality of tines (102, 102') extending therefrom dimensioned to engage and hold tissue when said jaw members are moved from said first position to said second position, wherein at least one of said plurality of tines has a free gripping end (122) being pointed; **characterised in that** is said first gripping device is disposed on at least one of said outer right and left side surfaces of one of said jaw members.

2. A bipolar electrosurgical instrument according to claim 1, wherein each of said plurality of tines has a free gripping end (122), said free gripping end being suitable to manipulate the epidermis section (12, 12') of tissue but not disturb the subcutaneous adipose tissue layer (16, 16') when said outer member and said inner member move from said first position to said second position.

3. A bipolar electrosurgical instrument according to claim 1 or 2 further comprising:
a second gripping device (100) disposed on the other of said at least one of said right and left side surfaces of the other of said jaw members, said second gripping device including said plurality of tines extending therefrom which cooperate with said first gripping device to engage and hold tissue when said jaw members are moved from said first position to said second position.

4. A bipolar electrosurgical instrument according to claim 3, wherein the plurality of tines disposed on one of the jaw members is offset relative to the plurality of tines disposed on the other jaw member along the length of the jaw members.

5. A bipolar electrosurgical instrument according to any one of the preceding claims, wherein each tine is a resilient member.

6. A bipolar electrosurgical instrument according to any one of the preceding claims, wherein at least one of said plurality of tines extends from said jaw member at an angle.

7. A bipolar electrosurgical instrument according to any one of the preceding claims, wherein each tine has a complementary size relative to a remainder of said plurality of tines, and wherein said plurality of tines are configured to form a gripping zone.

8. A bipolar electrosurgical instrument according to any one of claims 1 to 5, wherein said plurality of tines extend from said respective jaw member at an angle, said angle being measured relative to a horizontal axis of said jaw member, said horizontal axis being about perpendicular to said seal surface, said angle being about forty five degrees.

9. A bipolar electrosurgical instrument according to any one of the preceding claims, wherein each tine is straight.

10. A bipolar electrosurgical instrument according to any one of claims 1 to 9, wherein each tine is curved.

11. A bipolar electrosurgical instrument according to any one of the preceding claims, wherein each tine is a resilient member configured to move a predetermined amount of tissue from a separated position to another position between said seal surfaces.

12. A bipolar electrosurgical instrument according to any one of the preceding claims, wherein each of said plurality of tines is a rectangular member configured in a substantially "U" shape, with each tine being a complementary size relative to one another, said plurality of tines configured to form a gripping zone.

13. A bipolar electrosurgical instrument according to any one of claims 1 to 12, wherein each tine is a U shaped member including a base portion connected to said first gripping device.

14. A bipolar electrosurgical instrument according to any one of claims 1 to 13, wherein at least one of said plurality of tines has an end (122) with a plurality of points.

15. A bipolar electrosurgical instrument according to any one of the preceding claims, wherein at least one of said plurality of tines has a different end relative to another one of said plurality of tines.

16. A bipolar electrosurgical instrument according to any one of the preceding claims, comprising:
at least one tine disposed at a distal-most end of each of said jaw members in opposing relation thereto, said tines being dimensioned to engage and hold tissue when said jaw members are moved from said first position to said second position,
wherein said tines form an epidermis gripping zone, said epidermis gripping zone being suitable to grasp and retain the epidermis section (12, 12') of tissue and the dermis section (14, 14') of tissue between said seal surfaces but not contact any subcutaneous adipose tissue layer (16, 16').

## Patentansprüche

1. Bipolares elektrochirurgisches Instrument (20), mit:
einem inneren und einem äußeren Element (32', 32), jedes ein jeweiliges Backenelement (50, 60) aufweisend, das an dessen distalen Ende angeordnet ist, wobei jedes der Backenelemente eine äußere rechte und linke Seitenfläche (22, 22') und eine Versiegelungsfläche aufweist und die Versiegelungsflächen (24, 24') angepasst sind, sich mit einer elektrischen Energiequelle zu verbinden, sodass die Versiegelungsflächen imstande sind, zwischen sich bipolare Energie zu leiten;
wobei das innere und äußere Element von einer ersten Position, in der die Backenelemente relativ zueinander in einer beabstandeten Beziehung angeordnet sind, zu einer zweiten Position bewegbar sind, in der die Backenelemente zum Greifen von Gewebe näher aneinander sind; und
einer ersten Greifeinrichtung (100), die eine Vielzahl von Zinken (102, 102') aufweist, die sich von ihr erstrecken und dimensioniert sind, in Gewebe einzugreifen und es zu halten, wenn die Backenelemente von der ersten Position zu der zweiten Position bewegt werden, wobei mindestens eine der Vielzahl von Zinken ein freies Greifende (122) aufweist, das spitz ist, **dadurch gekennzeichnet, dass** die erste Greifeinrichtung an mindestens einer der äußeren rechten und linken Seitenfläche eines der Backenelemente angeordnet ist.

2. Bipolares elektrochirurgisches Instrument nach Anspruch 1, bei dem jede der Vielzahl von Zinken ein freies Greifende (122) aufweist, das geeignet ist, den Epidermisbereich (12, 12') des Gewebes zu manipulieren, aber nicht die subkutane adipöse Gewebeschicht (16, 16') zu stören, wenn sich das äußere Element und das innere Element von der ersten Position zu der zweiten Position bewegen.

3. Bipolares elektrochirurgisches Instrument nach Anspruch 1 oder 2, des Weiteren mit:
einer zweiten Greifeinrichtung (100), die an der anderen rechten und/oder linken Seitenfläche des anderen der Backenelemente angeordnet ist, wobei die zweite Greifeinrichtung die Vielzahl der sich von ihr erstreckenden Zinken aufweist, die mit der ersten Greifeinrichtung zusammenwirken, um in Gewebe einzugreifen und es zu halten, wenn die Backenelemente von der ersten Position zu der zweiten Position bewegt werden.

4. Bipolares elektrochirurgisches Instrument nach Anspruch 3, bei dem die Vielzahl von Zinken, die an einem der Backenelemente angeordnet sind, relativ zu der Vielzahl von Zinken, die an dem anderen Backenelement angeordnet sind, versetzt sind, und zwar entlang der Länge der Backenelemente.

5. Bipolares elektrochirurgisches Instrument nach einem der vorstehenden Ansprüche, bei dem jede Zinke ein Federelement ist.

6. Bipolares elektrochirurgisches Instrument nach einem der vorstehenden Ansprüche, bei dem mindestens eine der Vielzahl von Zinken sich von dem Backenelement in einem Winkel erstreckt.

7. Bipolares elektrochirurgisches Instrument nach einem der vorstehenden Ansprüche, bei dem jede Zinke eine komplementäre Größe relativ zu einem Rest der Vielzahl von Zinken aufweist und bei dem die Vielzahl von Zinken eingerichtet sind, eine Greifzone auszubilden.

8. Bipolares elektrochirurgisches Instrument nach einem der Ansprüche 1 bis 5, bei dem sich die Vielzahl von Zinken von dem jeweiligen Backenelement in einem Winkel erstrecken, der relativ zu einer horizontalen Achse des Backenelements gemessen wird, die in etwa senkrecht zu der Versiegelungsfläche ist, wobei der Winkel in etwa 45 Grad beträgt.

9. Bipolares elektrochirurgisches Instrument nach einem der vorstehenden Ansprüche, bei dem jede Zinke gerade ist.

10. Bipolares elektrochirurgisches Instrument nach einem der Ansprüche 1 bis 9, bei dem jede Zinke gekrümmt ist.

11. Bipolares elektrochirurgisches Instrument nach einem der vorstehenden Ansprüche, bei dem jede Zinke ein Federelement ist, das eingerichtet ist, eine festgelegte Gewebemenge von einer getrennten Position zu einer anderen Position zwischen den Versiegelungsflächen zu bewegen.

12. Bipolares elektrochirurgisches Instrument nach einem der vorstehenden Ansprüche, bei dem jede der Vielzahl von Zinken ein rechtwinkliges Element ist, das im Wesentlichen als U-Form eingerichtet ist, und zwar mit jeder Zinke relativ zu einer anderen eine komplementäre Größe aufweisend, wobei die Vielzahl von Zinken eingerichtet ist, eine Greifzone auszubilden.

13. Bipolares elektrochirurgisches Instrument nach einem der Ansprüche 1 bis 12, bei dem jede Zinke ein U-förmiges Element ist, das einen Basisabschnitt aufweist, der mit der ersten Greifeinrichtung verbunden ist.

14. Bipolares elektrochirurgisches Instrument nach einem der Ansprüche 1 bis 13, bei dem mindestens eine der Vielzahl von Zinken ein Ende (122) mit einer Vielzahl von Spitzen aufweist.

15. Bipolares elektrochirurgisches Instrument nach einem der vorstehenden Ansprüche, bei dem mindestens eine der Vielzahl von Zinken ein relativ zu einer anderen der Vielzahl von Zinken unterschiedliches Ende aufweist.

16. Bipolares elektrochirurgisches Instrument nach einem der vorstehenden Ansprüche, mit:
mindestens einer Zinke, die an einem äußersten distalen Ende eines jeden Backenelements in gegenüberstehender Beziehung dazu angeordnet ist, und die Zinken dimensioniert sind, in Gewebe einzugreifen und es zu halten, wenn die Backenelemente von der ersten Position zu der zweiten Position bewegt werden,
wobei die Zinken eine Epidermis-Greifzone ausbilden, die geeignet ist, den Epidermisbereich (12, 12') des Gewebes und den Dermisbereich (14, 14') des Gewebes zwischen den Versiegelungsflächen zu greifen und zu halten, aber keine subkutane adipöse Gewebeschicht (16, 16') zu berühren.

## Revendications

1. Instrument électrochirurgical bipolaire (20) comprenant:
des éléments intérieur et extérieur (32', 32) ayant chacun un élément de mâchoire respectif (50, 60) disposé à son extrémité distale, chacun desdits éléments de mâchoire comportant des surfaces latérales extérieures droite et gauche (22, 22') et une surface de suture, lesdites surfaces de suture (24, 24') étant aptes à être connectées à une source d'énergie électrique de telle sorte que les surfaces de suture sont aptes à conduire l'énergie bipolaire entre elles;
lesdits éléments intérieur et extérieur sont déplaçables d'une première position, dans laquelle lesdits éléments de mâchoire sont disposés en une relation espacée l'un relativement à l'autre à une seconde position dans laquelle les éléments de mâchoire sont plus proches l'un de l'autre pour saisir le tissu; et
un premier dispositif de préhension (100) incluant une pluralité de dents (102, 102') s'étendant de celui-ci, dimensionnées pour venir en prise et tenir le tissu lorsque lesdits éléments de mâchoire sont déplacés de ladite première position à ladite seconde position, où au moins une de ladite pluralité de dents possède une extrémité de préhension libre (122) qui est pointue;
**caractérisé en ce que** ledit premier dispositif de préhension est disposé sur au moins une desdites surfaces latérales extérieures droite et gauche d'un desdits éléments de mâchoire.

2. Instrument électrochirurgical bipolaire selon la revendication 1, dans lequel chacune de ladite pluralité de dents possède une extrémité de préhension libre (122), ladite extrémité de préhension libre étant apte à manipuler une section d'épiderme (12, 12') de tissu sans perturber la couche de tissu d'adipocyte sous-cutanée (16, 16') lorsque ledit élément extérieur et ledit élément intérieur passent de ladite première position à ladite seconde position.

3. Instrument électrochirurgical bipolaire selon la revendication 1 ou 2, comprenant en outre:
un deuxième dispositif de préhension (100) disposé sur l'autre desdites au moins une surface latérale droite et gauche de l'autre desdits éléments de mâchoire, ledit deuxième dispositif de préhension incluant ladite pluralité de dents s'étendant de celui-ci qui coopèrent avec ledit premier dispositif de préhension pour venir en prise avec et tenir le tissu lorsque lesdits éléments de mâchoire sont déplacés de ladite première position à ladite seconde position.

4. Instrument électrochirurgical bipolaire selon la revendication 3, où la pluralité de dents disposées sur un des éléments de mâchoire est décalée relativement à la pluralité de dents disposées sur l'autre élément de mâchoire sur la longueur des éléments de mâchoire.

5. Instrument électrochirurgical bipolaire selon l'une quelconque des revendications précédentes, où chaque dent est un élément résiliant.

6. Instrument électrochirurgical bipolaire selon l'une quelconque des revendications précédentes, où au moins une de ladite pluralité de dents s'étend dudit élément de mâchoire selon un angle.

7. Instrument électrochirurgical bipolaire selon l'une quelconque des revendications précédentes, où chaque dent a une taille complémentaire relativement à un reste de ladite pluralité de dents, et où ladite pluralité de dents sont configurées pour former une zone de préhension.

8. Instrument électrochirurgical bipolaire selon l'une quelconque des revendications 1 à 5, où ladite pluralité de dents s'étendent dudit élément de mâchoire respectif selon un angle, ledit angle étant mesuré relativement à un axe horizontal dudit élément de mâchoire, ledit axe horizontal étant environ perpendiculaire à ladite surface de suture, ledit angle étant d'environ quarante cinq degrés.

9. Instrument électrochirurgical bipolaire selon l'une quelconque des revendications précédentes, où chaque dent est droite.

10. Instrument électrochirurgical bipolaire selon l'une quelconque des revendications 1 à 9, où chaque dent est courbée.

11. Instrument électrochirurgical bipolaire selon l'une quelconque des revendications précédentes, où chaque dent est un élément résiliant configuré pour déplacer une quantité de tissu prédéterminée d'une position séparée à une autre position entre lesdites surfaces de suture.

12. Instrument électrochirurgical bipolaire selon l'une quelconque des revendications précédentes, où chacune de ladite pluralité de dents est un élément rectangulaire réalisé en une forme sensiblement en "U", chaque dent ayant une taille complémentaire relativement à l'autre, ladite pluralité de dents étant configurée pour former une zone de préhension.

13. Instrument électrochirurgical bipolaire selon l'une quelconque des revendications 1 à 12, où chaque dent est un élément en forme de U comportant une portion de base reliée audit premier dispositif de préhension.

14. Instrument électrochirurgical bipolaire selon l'une quelconque des revendications 1 à 13, où au moins une de ladite pluralité de dents possède une extrémité (122) avec une pluralité de pointes.

15. Instrument électrochirurgical bipolaire selon l'une quelconque des revendications précédentes, où au moins une de ladite pluralité de dents a une extrémité différente relativement à une autre de ladite pluralité de dents.

16. Instrument électrochirurgical bipolaire selon l'une quelconque des revendications précédentes, comprenant:
au moins une dent disposée à une extrémité la plus distale de chacun desdits éléments de mâchoire en une relation opposée à ceux-ci, lesdites dents étant dimensionnées pour venir en prise avec et tenir le tissu lorsque lesdits éléments de mâchoire sont amenés de ladite première position à ladite seconde position,
où lesdites dents forment une zone de préhension d'épiderme, ladite zone de préhension d'épiderme étant apte à saisir et à retenir la section d'épiderme (12, 12') de tissu, et la section de derme (14, 14') de tissu entre lesdites surfaces de suture mais sans venir en contact avec la couche de tissu d'adipocyte sous-cutanée (16, 16').
